# EUROPEAN PATENT APPLICATION

(11) **EP 3 058 895 A1**
(43) Date of publication of application: **24.08.2016**
(21) Application number: 16154345.9
(22) Date of filing: 04.02.2016
(51) Int. Cl.: A61F 2/24

(54) **INTRODUCER SHEATH FOR TRANSCATHETER HEART VALVE DELIEVERY**

(30) Priority: 18.02.2015 US 201562117630 P
(71) Applicant: St. Jude Medical, Cardiology Division, Inc., St. Paul MN 55117 (US)
(72) Inventor: THOMAS, Ralph Joseph, Champlin, MN 55316 (US); GEIGER, Gary W., Fridley, MN 55432 (US)
(74) Representative: Gill Jennings & Every LLP

(57) **Abstract**

An introducer sheath includes a tubular cannula extending between a leading end and trailing end, the cannula defining a lumen therethrough. A first balloon may be disposed adjacent the leading end of the cannula, the first balloon being configured and arranged to dilate from a collapsed condition to an expanded condition. An inflation port may be configured to receive an inflation medium to dilate the first balloon.

## Description

### CROSS-REFERENCE TO RELATED APPLICATIONS

The present application claims the benefit of the filing date of U.S. Provisional Patent Application No. 62/117,630 filed February 18, 2015, the disclosure of which is hereby incorporated herein by reference.

### BACKGROUND OF THE INVENTION

The present invention is related to prosthetic heart valve replacement, and more particularly to devices, systems, and methods for transcatheter delivery of collapsible prosthetic heart valves.

Prosthetic heart valves that are collapsible to a relatively small circumferential size can be delivered into a patient less invasively than valves that are not collapsible. For example, a collapsible valve may be delivered into a patient via a tube-like delivery apparatus such as a catheter, a trocar, a laparoscopic instrument, or the like. This collapsibility can avoid the need for a more invasive procedure such as full open-chest, open-heart surgery.

Collapsible prosthetic heart valves typically take the form of a valve structure mounted on a stent. There are two types of stents on which the valve structures are ordinarily mounted: a self-expanding stent and a balloon-expandable stent. To place such valves into a delivery apparatus and ultimately into a patient, the valve must first be collapsed or crimped to reduce its circumferential size.

When a collapsed prosthetic valve has reached the desired implant site in the patient (*e.g.,* at or near the annulus of the patient's heart valve that is to be replaced by the prosthetic valve), the prosthetic valve can be deployed or released from the delivery apparatus and re-expanded to full operating size. For balloon-expandable valves, this generally involves releasing the entire valve, assuring its proper location, and then expanding a balloon positioned within the valve stent. For self-expanding valves, on the other hand, the stent automatically expands as the sheath covering the valve is withdrawn.

Despite the various improvements that have been made to the collapsible prosthetic heart valve delivery process, conventional delivery devices, systems, and methods suffer from some shortcomings. For example, in conventional delivery devices for self-expanding valves, it may be difficult to introduce the delivery device into the body. Specifically, it may be difficult to simultaneously manipulate a delivery device and an introducer sheath while keeping the introducer sheath in the correct position.

There therefore is a need for further improvements to the devices, systems, and methods for transcatheter delivery of collapsible prosthetic heart valves, and in particular, the introduction of such prosthetic heart valves into the heart. Among other advantages, the present invention may address one or more of these needs.

### BRIEF SUMMARY OF THE INVENTION

In some embodiments, an introducer sheath includes a tubular cannula extending between a leading end and trailing end, the cannula defining a lumen therethrough, a first balloon disposed adjacent the leading end of the cannula, the first balloon being configured and arranged to dilate from a collapsed condition to an expanded condition, and an inflation port configured to receive an inflation medium to dilate the first balloon.

In some embodiments, a method of delivering a collapsible prosthetic heart valve includes piercing an insertion location of a patient's body, partially inserting an introducer sheath into the patient's body, the insertion sheath having (i) a tubular cannula extending between a leading end and trailing end, the cannula defining a lumen therethrough, (ii) a first balloon disposed adjacent the leading end of the cannula, the first balloon being configured and arranged to dilate from a collapsed condition to an expanded condition, and (iii) an inflation port configured to receive an inflation medium to dilate the first balloon, and dilating the first balloon inside the patient's body to prevent movement of the introducer sheath in at least one direction.

### BRIEF DESCRIPTION OF THE DRAWINGS

Various embodiments of the present invention will now be described with reference to the appended drawings. It is to be appreciated that these drawings depict only some embodiments of the invention and are therefore not to be considered limiting of its scope.
FIG. 1A is a top plan view of a portion of an operating handle of a delivery device for a collapsible prosthetic heart valve, shown with a partial longitudinal cross-section of the distal portion of a catheter assembly;
FIG. 1B is a side view of the handle of FIG. 1A;
FIG. 2 is a side elevational view of a conventional prosthetic heart valve;
FIG. 3A is a side view of an introducer for a delivery device having a balloon;
FIG. 3B is a side view of the introducer of FIG. 3A after the balloon has been inflated;
FIG. 4 is a highly schematic illustration showing the use of an introducer sheath for transcatheter valve replacement using a transaortic approach;
FIG. 5 is a highly schematic illustration showing the use of an introducer sheath for transcatheter valve replacement using a transapical approach; and
FIG. 6 is a side view of a second example of an introducer for a delivery device having two balloons.

### DETAILED DESCRIPTION

As used herein, the terms "proximal," "distal," "leading" and "trailing" are to be taken as relative to a user using the disclosed delivery devices. "Proximal" or "trailing end" are to be understood as relatively close to the user and "distal" or "leading end" are to be understood as relatively farther away from the clinician. Also, as used herein, the words "substantially," "approximately," "generally" and "about" are intended to mean that slight variations from absolute are included within the scope of the structure or process recited.

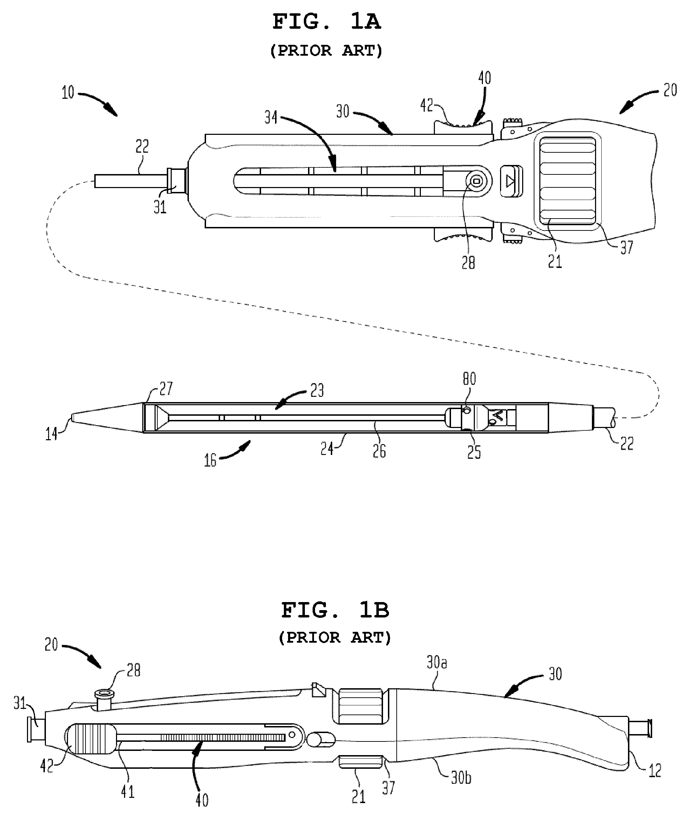

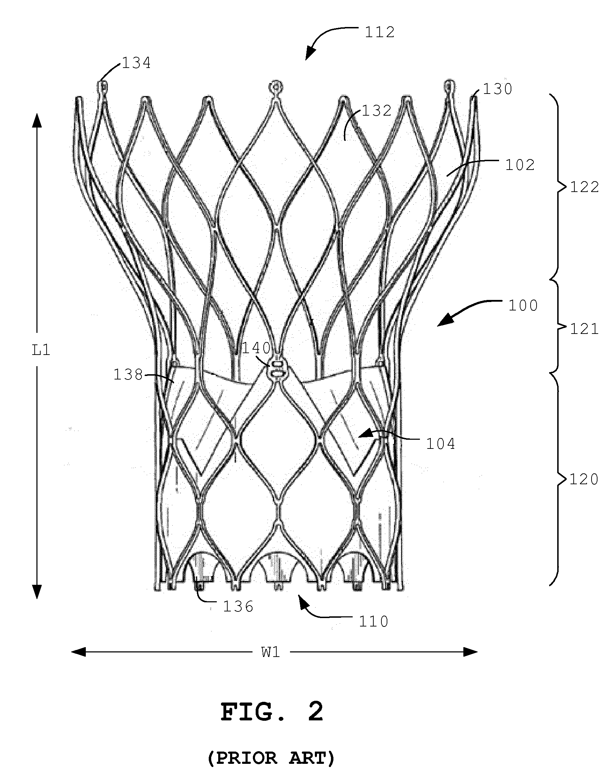

Referring now to FIGS. 1A and 1B, the structure and function of a transaortic or transfemoral delivery device will be described. It will be understood, however, that the devices and methods disclosed herein also may be used with a transapical or transseptal delivery device. An exemplary transaortic delivery device 10 is shown for delivering a prosthetic heart valve, which will be described in greater detail below with reference to Fig. 2. Transaortic delivery device 10 has a catheter assembly 16 for delivering the heart valve to and deploying the heart valve at a target location, and an operating handle 20 for controlling deployment of the valve from the catheter assembly. Delivery device 10 extends from a proximal end 12 to an atraumatic tip 14 at the distal end of catheter assembly 16. Catheter assembly 16 is adapted to receive a collapsible prosthetic heart valve (not shown) in a compartment 23 defined around an inner shaft 26 and covered by a distal sheath 24.

Inner shaft 26 may extend from operating handle 20 to atraumatic tip 14 of the delivery device, and may include a retainer 25 affixed thereto at a spaced distance from tip 14 and adapted to hold a collapsible prosthetic valve in compartment 23. Retainer 25 may have recesses 80 therein that are adapted to hold corresponding retention members of the valve. Inner shaft 26 may be made of a flexible material such as braided polyimide or polyetheretherketone (PEEK), for example. Using a material such as PEEK may improve the resistance of inner shaft 26 to kinking while catheter assembly 16 is tracking through the vasculature of a patient.

Distal sheath 24 surrounds inner shaft 26 and is slidable relative to the inner shaft such that it can selectively cover or uncover compartment 23. Distal sheath 24 is affixed at its proximal end to an outer shaft 22, the proximal end of which is connected to operating handle 20 in a manner to be described. Distal end 27 of distal sheath 24 abuts atraumatic tip 14 when the distal sheath is fully covering compartment 23, and is spaced apart from the atraumatic tip when compartment 23 is at least partially uncovered.

Operating handle 20 is adapted to control deployment of a prosthetic valve located in compartment 23 by permitting a user to selectively slide outer shaft 22 proximally or distally relative to inner shaft 26, thereby respectively uncovering or covering the compartment with distal sheath 24. Outer shaft 22 may be made of a flexible material such as nylon 11 or nylon 12, and it may have a round braid construction (*i.e.,* round cross-section fibers braided together) or flat braid construction (*i.e.,* rectangular cross-section fibers braided together), for example.

The proximal end of inner shaft 26 may be connected in a substantially fixed relationship to an outer housing 30 of operating handle 20, and the proximal end of the outer shaft 22 may be affixed to a carriage assembly 40 that is slidable along a longitudinal axis of the handle housing, such that a user can selectively slide the outer shaft relative to the inner shaft by sliding the carriage assembly relative to the housing. Operating handle 20 may further include a hemostasis valve 28 having an internal gasket adapted to create a seal between inner shaft 26 and the proximal end of outer shaft 22.

As shown, handle housing 30 includes a top portion 30a and a bottom portion 30b. Top and bottom portions 30a and 30b may be individual pieces joined to one another as shown in FIG. 1B. Collectively, top and bottom portions 30a and 30b define an elongated space 34 in housing 30 in which carriage assembly 40 may travel. Optionally, top and bottom portions 30a and 30b may further form a substantially cylindrical boss 31 for accepting a clip, as will be described below. Elongated space 34 preferably permits carriage assembly 40 to travel a distance that is at least as long as the anticipated length of the prosthetic valve to be delivered (*e.g.,* at least about 50 mm), such that distal sheath 24 can be fully retracted from around the prosthetic valve. Carriage assembly 40 includes a pair of carriage grips 42, each attached to a body portion 41. Although the carriage assembly 40 is shown in FIGS. 1A and 1B as having two carriage grips 42, that need not be the case.

Handle housing 30 further defines a pocket 37 that extends through top portion 30a and bottom portion 30b for receiving a deployment actuator 21. Pocket 37 is sized and shaped to receive deployment actuator 21 with minimal clearance, such that the location of deployment actuator remains substantially fixed relative to housing 30 as it is rotated. Deployment actuator 21 may be internally coupled to body portion 41 via a threaded shaft or other suitable connection such that rotation of the deployment actuator in one direction (either clockwise or counterclockwise) pulls the body portion 41 of carriage assembly 40 proximally through elongated space 34.

To use operating handle 20 to deploy a prosthetic valve that has been loaded into compartment 23 and covered by distal sheath 24, the user may rotate deployment actuator 21, causing carriage assembly 40 to slide proximally within elongated space 34 in housing 30. Because distal sheath 24 is affixed to outer shaft 22, which in turn is affixed to carriage assembly 40, and because inner shaft 26 is fixed to housing 30, sliding the carriage assembly proximally relative to the housing will retract the distal sheath proximally from compartment 23, thereby exposing and initiating deployment of the valve located therein.

Delivery device 10 may be used to implant a medical device such as a collapsible stent-supported prosthetic heart valve 100 having a stent 102 and a valve assembly 104 (FIG. 2). The prosthetic heart valve 100 is designed to replace a native tricuspid valve of a patient, such as a native aortic valve. It should be noted that while the devices disclosed herein are described predominantly in connection with their use with a prosthetic aortic valve and a stent having a shape as illustrated in Fig. 2, the valve could be a bicuspid or other valve, such as the mitral valve, and the stent could have different shapes, such as a flared or conical annulus section, a less-bulbous aortic section, and the like, and a differently shaped transition section.

The expandable stent 102 of prosthetic heart valve 100 may be formed from biocompatible materials that are capable of self-expansion, such as, for example, shape memory alloys, such as the nickel-titanium alloy known as "nitinol," or other suitable metals or polymers. Stent 102 extends in a length direction L1 from proximal or annulus end 110 to distal or aortic end 112, and includes annulus section 120 adjacent proximal end 110, transition section 121, and aortic section 122 adjacent distal end 112. Annulus section 120 has a relatively small cross-section in the expanded condition, while aortic section 122 has a relatively large cross-section in the expanded condition. Preferably, annulus section 120 is in the form of a cylinder having a substantially constant diameter along its length. Transition section 121 may taper outwardly from annulus section 120 to aortic section 122. Each of the sections of stent 102 includes a plurality of struts 130 forming cells 132 connected to one another in one or more annular rows around the stent. For example, as shown in Fig. 2, annulus section 120 may have two annular rows of complete cells 132 and aortic section 122 and transition section 121 may each have one or more annular rows of partial cells 132. Cells 132 in aortic section 122 may be larger than cells 132 in annulus section 120. The larger cells in aortic section 122 better enable prosthetic valve 100 to be positioned in the native valve annulus without the stent structure interfering with blood flow to the coronary arteries. Each of cells 132 has a length in length direction L1 of the stent and a width W1 in a perpendicular direction.

Stent 102 may include one or more retaining elements 134 at distal end 112 thereof, retaining elements 134 being sized and shaped to cooperate with female retaining structures (not shown) provided on a deployment device. The engagement of retaining elements 134 with the female retaining structures on the deployment device helps maintain prosthetic heart valve 100 in assembled relationship with the deployment device, minimizes longitudinal movement of the prosthetic heart valve relative to the deployment device during unsheathing or resheathing procedures, and helps prevent rotation of the prosthetic heart valve relative to the deployment device as the deployment device is advanced to the target location and the heart valve deployed.

Valve assembly 104 of prosthetic heart valve 100 preferably is positioned in annulus section 120 of stent 102 and secured to the stent. Valve assembly 104 includes cuff 136 and a plurality of leaflets 138 which collectively function as a one-way valve by coapting with one another. As a prosthetic aortic valve, valve 100 has three leaflets 138.

Although cuff 136 is shown in Fig. 2 as being disposed on the luminal or inner surface of annulus section 120, it is contemplated that cuff 136 may be disposed on the abluminal or outer surface of annulus section 120 or may cover all or part of either or both of the luminal and abluminal surfaces. Both cuff 136 and leaflets 138 may be wholly or partly formed of any suitable biological material or polymer such as, for example, polytetrafluoroethylene (PTFE).

Leaflets 138 may be attached along their belly portions to cells 132 of stent 102, with the commissure between adjacent leaflets 138 attached to a commissure feature 140. As can be seen in Fig. 2, each commissure feature 140 may lie at the intersection of four cells 132, two of the cells being adjacent one another in the same annular row, and the other two cells being in different annular rows and lying in end-to-end relationship. Preferably, commissure features 140 are positioned entirely within annulus section 120 or at the juncture of annulus section 120 and transition section 121. Commissure features 140 may include one or more eyelets which facilitate the suturing of the leaflet commissure to stent 102.

Prosthetic heart valve 100 may be used to replace a native aortic valve, a surgical heart valve or a heart valve that has undergone a surgical procedure. Prosthetic heart valve 100 may be delivered to the desired site (*e.g.,* near the native aortic annulus) using any suitable delivery device. During delivery, prosthetic heart valve 100 is disposed inside the delivery device in the collapsed condition. The delivery device may be introduced into a patient using a transfemoral, transapical, transseptal or any other percutaneous approach. Once the delivery device has reached the target site, the user may deploy prosthetic heart valve 100. Upon deployment, prosthetic heart valve 100 expands so that annulus section 120 is in secure engagement within the native aortic annulus. When prosthetic heart valve 100 is properly positioned inside the heart, it works as a one-way valve, allowing blood to flow from the left ventricle of the heart to the aorta, and preventing blood from flowing in the opposite direction.

As briefly discussed, several approaches are possible to introduce the delivery device into the patient. With a transfemoral approach, the delivery device is introduced into the transfemoral artery of the patient. With transaortic and transapical approaches, shorter paths are taken to the aortic valve through the patient's chest. In such cases, an introducer sheath may be useful to advance the delivery device to the target location.

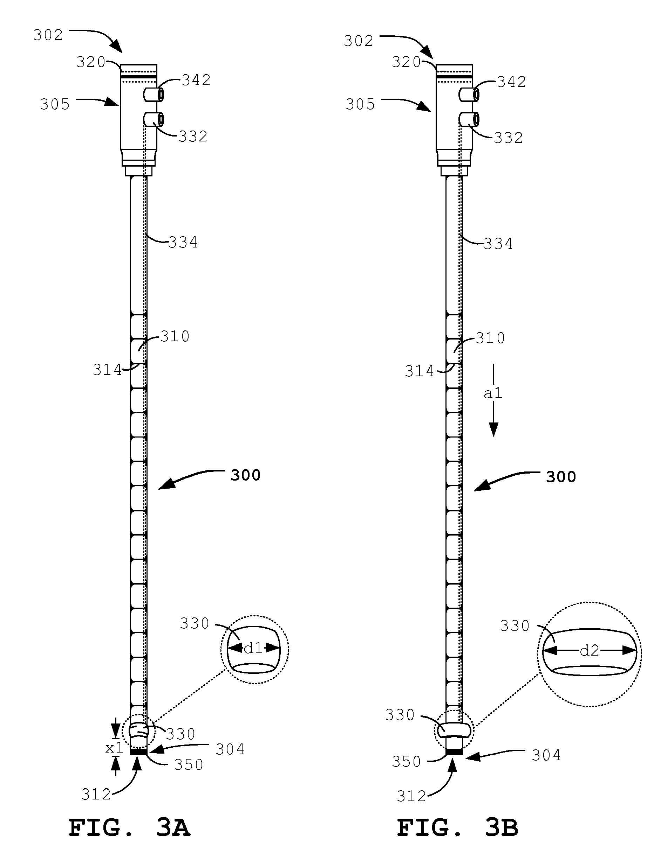

FIG. 3A illustrates introducer sheath 300, which extends between trailing end 302 and leading end 304. Introducer sheath 300 includes hub 305 disposed adjacent trailing end 302 and cannula 310 extending from hub 305 to leading end 304. Hub 305 and cannula 310 may be hollow to define lumen 312 between trailing end 302 and leading end 304. In at least some examples, cannula 310 may include lumen 312 having an inner diameter of approximately 16 French. In at least some examples, the inner diameter of lumen 312 may be between about 17 French and about 20 French and able to accommodate delivery systems of varying diameters. Cannula 310 includes a plurality of markings 314 on its abluminal surface, markings 314 indicating certain distances along the length of cannula 310. In at least some examples, markings 314 are spaced between approximately 0.25 and approximately 0.5 inches apart to indicate the depth of cannula 310 during use. Hemostasis seal 320 may also be disposed on the interior of hub 305 and configured to prevent backflow of liquid through lumen 312. In some examples, hemostasis seal 320 is configured to prevent blood loss through introducer sheath 300 during the operation of the device.

Introducer sheath 300 further includes balloon 330 disposed adjacent leading end 304 and fixed at a predetermined distance x1 from leading end 304 along cannula 310. In at least some examples distance x1 is between approximately 1.0 mm and approximately 30.0 mm. Introducer sheath 300 further includes inflation port 332 disposed on hub 305 and inflation tube 334 extending between inflation port 332 and balloon 330. Inflation tube 334 may be in communication with the interior of balloon 330 and configured to deliver an inflation media to the interior of balloon 330 to expand the balloon from a collapsed condition to an expanded condition. In at least some examples, the inflation media includes a liquid, such as a saline solution, contrast media, or a bioabsorbable gas such as nitrogen. As shown in FIGS. 3A and 3B, hub 305 may further include flush port 342 for flushing the interior of cannula 310 (i.e. lumen 312). Saline may also be used to flush lumen 312 to remove air and lubricate the interior of cannula 310 for smooth delivery. Moreover, to aid in placement, one or more radiopaque markers 350 may be disposed on the leading end 304 of introducer sheath 300. In at least some examples, radiopaque marker 350 is disposed adjacent balloon 330.

FIG. 3B illustrates introducer sheath 300 after inflation of the balloon at the leading end 304 of the device. As shown in FIG. 3B, inflation media is introduced inflation port 332 and travels through inflation tube 334 in the direction indicated by arrow "a1." As media fills balloon 330, the balloon circumferentially swells to its expanded condition as shown in Fig. 3B. While certain dimensions are provided herein, it will be understood that such dimensions are merely exemplary and not limiting. In at least some examples, balloon 330 has a first diameter d1 of approximately 6.9 mm (0.273 inches) to 7.1 mm (0.28 inches) in the collapsed condition and a second diameter d2 of approximately 7.1 mm (0.28 inches) to 30 mm (1.18 inches) in the fully expanded condition. In at least some examples, diameter d1 is not much larger than the diameter of the cannula itself so that the balloon can easily pass through the insertion point in the collapsed condition. In such examples, diameter d1 of balloon 330 in the collapsed condition is approximately less than 0.5 mm (0.020 inches) inches greater than the diameter of cannula 310. After expansion, however, diameter d2 may be approximately 1.0 mm (0.039 inches) to 23 mm (0.90 inches) greater than the diameter of cannula 310.

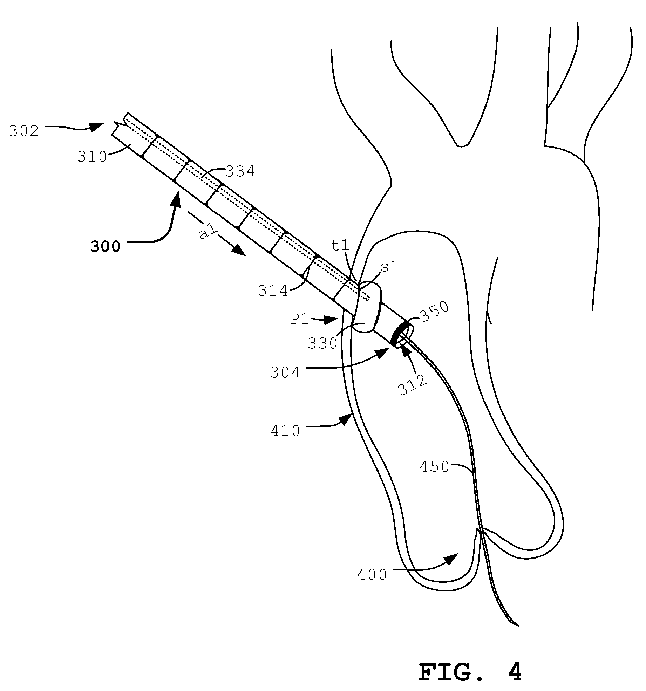

Fig. 4 illustrates a transaortic approach using introducer sheath 300 to aid in advancing a delivery device to a native heart annulus 400. A transaortic approach may be preferable to a transfemoral approach under certain conditions and clinical settings. For example, patients with severe pulmonary disease as well as patients with narrowed arteries may be better suitable for a transaortic approach.

As seen in Fig. 4, an incision is made in ascending aorta 410 at puncture location P1. A dilator (not shown) having an increasing diameter may be used to widen the incision at puncture location P1. Guidewire 450 may be advanced in retrograde fashion from puncture location P1, down ascending aorta 410 through native valve annulus 400. Introducer sheath 300 may then be advanced over guidewire 450 from puncture location P1 toward native valve annulus 400. Markings 314 may aid the clinician in determining the depth of cannula 310 with respect to ascending aorta 410. For example, cannula 310 may be advanced through puncture location P1 until a specific marking (e.g., the second marking) or a specific depth (e.g., 3 cm) is reached. Additionally, radiopaque marker 350 and/or other echogenic materials may be used to guide introducer sheath 300 to the appropriate position using the assistance of three-dimensional echocardiography to visualize the therapeutic device within the patient. Alternative visualization techniques known in the art are also contemplated herein.

With introducer sheath 300 at the desired depth and position, inflation media may be introduced through the inflation port (not shown) through inflation tube 334 to dilate balloon 330. After balloon 330 expands, upper surface s1 of balloon 330 contacts a portion of the patient's heart tissue t1 adjacent puncture location P1 so that cannula 310 can no longer freely travel through puncture location P1 due to the increased diameter of balloon 330. Thus, though cannula 310 is pulled back toward the clinician, it does not travel any further once balloon 330 abuts puncture location P1, preventing the accidental removal of introducer sheath 300 during the procedure. Delivery device (not shown) may then be advanced through lumen 312 of introducer sheath 300 and the procedure may continue with introducer sheath 300 safely secured at the proper position. Upon completion of the procedure, balloon 330 may be deflated by removing the inflation medium (e.g., nitrogen), thereby reducing the diameter of the balloon 330 such that the balloon is capable of fitting through puncture location P1. Introducer sheath 300 may then be withdrawn.

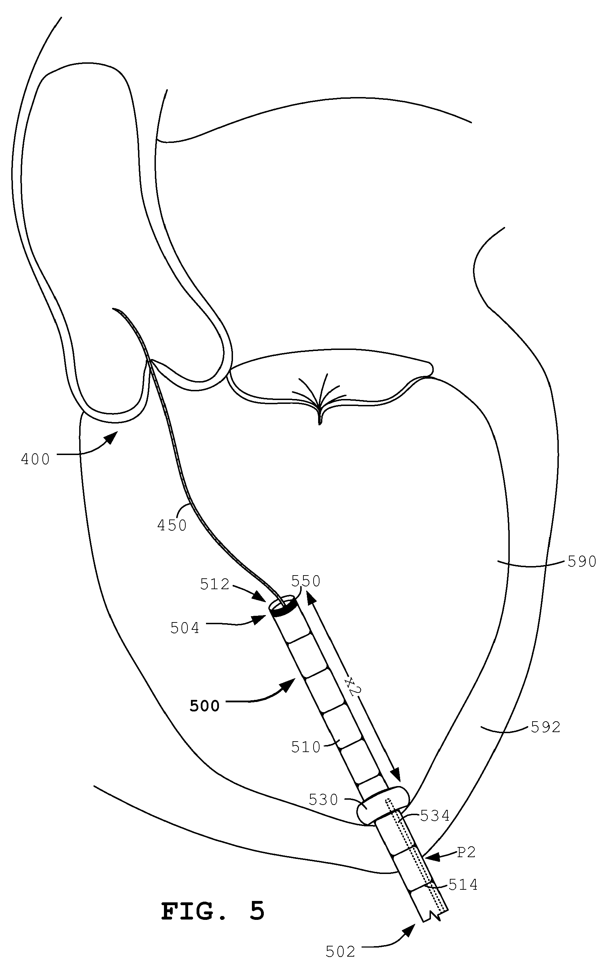

Fig. 5 illustrates a second approach, this time a transapical approach, using introducer sheath 500 to aid in advancing a delivery device to a native heart annulus 400. Introducer sheath 500 generally incorporates similar components to introducer sheath 300, which extends between trailing end 502 and leading end 504 and includes a hub (not shown) disposed adjacent trailing end 502 and cannula 510 extending from the hub to leading end 504. Hub 505 and cannula 510 define lumen 512 between trailing end 502 and leading end 504. Cannula 510 includes a plurality of markings 514 on its abluminal surface to indicate depth and balloon 530 disposed adjacent leading end 504 and fixed at a predetermined location along cannula 510. As seen in Fig. 5, in an introducer sheath for transapical applications, balloon 530 is fixed at a predetermined distance x2 from leading end 504 that is different than predetermined distance x1 of introducer sheath 300. Specifically, distance x2 is larger than distance x1 such that balloon 530 is disposed further away from leading end 504 in introducer sheath 500 for transapical applications. Introducer sheath 500 may further include markers 550, an inflation port (not shown), an inflation tube (not shown) and a flush port (also not shown) as described above.

In transapical applications, an incision is made in the heart at puncture location P2 adjacent left ventricle 590 to create an opening across heart wall 592. A dilator (not shown) having an increasing diameter may be used to widen the incision at puncture location P2. Guidewire 450 may be advanced from puncture location P2 toward native valve annulus 400. Introducer sheath 500 may then be advanced over guidewire 450 from puncture location P1 toward native valve annulus 400. Markings 514 may aid the clinician in determining the depth of cannula 510 with respect to native valve annulus 400.

With introducer sheath 500 at the desired depth and position, inflation media may be introduced through the inflation port (not shown) through the inflation tube (also not shown) to dilate balloon 530. After balloon 530 expands, cannula 510 can no longer freely travel through puncture location P2 due to the increased diameter of balloon 530. Thus, though cannula 510 is pulled back toward the clinician, it does not travel any further once balloon 530 abuts puncture location P2, preventing the accidental removal of introducer sheath 500 during the procedure. The procedure may then continue as desired. Upon completion of the procedure, balloon 530 may be deflated by removing the inflation medium (e.g., nitrogen), thereby reducing the diameter of the balloon 530 such that the balloon is capable of fitting through puncture location P2. Introducer sheath 500 may then be withdrawn.

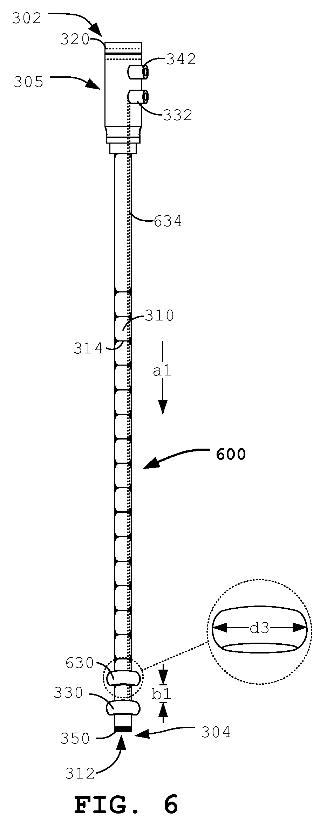

In another example, shown in Fig. 6, introducer sheath 600 generally includes all the elements of introducer sheath 300 (e.g., hub 305, cannula 310, markings 314, hemostasis seal 320, balloon 330, inflation port 332, flush port 342, and radiopaque marker 350). As shown, introducer sheath 600 further includes second balloon 630, spaced from balloon 330. Balloons 330 and 630 may be inflated by a single inflation medium through a single inflation port 332 via inflation tube 634. Inflation tube 634 may be configured to be in communication with the interiors of both balloons 330,630 such that the inflation medium delivered through inflation tube 634 in direction of arrow a1 dilates both balloons. Alternatively, two independent inflation ports may be disposed on hub 305, each port being in communication with a respective inflation tube to dilate a single balloon. In this example, balloons 330,630 may be independently dilated or deflated, sequentially or in concert, as desired. As shown, balloon 630 may be capable of dilating to a predetermined diameter d3, which is equal to or different than the dilated diameter d2 of balloon 330. In some examples, balloons 330,630 are spaced apart by a predetermined distance b1. Distance b1 may be approximately equal to the thickness of the wall of the aorta or the wall the left ventricle. Thus, inflating balloons 330,630 serves to anchor introducer sheath 600 at a predetermined position with respect to the native valve annulus.

It will be understood that various modification may be made to the disclosed embodiments without departing from the spirit of the disclosure. For example, introducer sheath may be used to introduce a delivery device into the heart for prosthetic heart valve replacement, or may be used to introduce devices for valve repair at any of the heart valve (e.g., aortic valve, mitral valve, pulmonary valve, tricuspid valve). Additionally, introducer sheath may be used to deliver instruments to repair other structures in the heart, such as the chordae tendineae, papillary muscles and the like. Introducer sheath may also be used to deliver embolism prevention devices and stents, grafts and other cardiovascular devices. Introducer sheath may also be used to introduce any other medical instruments or device into a patient's body in applications other than cardiovascular applications and may be useful in any bodily location where temporarily affixing a sheath a certain distance from body tissue is useful.

In some embodiments, an introducer sheath includes a tubular cannula extending between a leading end and trailing end, the cannula defining a lumen therethrough, a first balloon disposed adjacent the leading end of the cannula, the first balloon being configured and arranged to dilate from a collapsed condition to an expanded condition, and an inflation port configured to receive an inflation medium to dilate the first balloon.

In some examples, the sheath may include a plurality of spaced markings disposed on the abluminal surface of the cannula; and/or the first balloon may be configured to circumferentially dilate from a first diameter in the collapsed condition to a second diameter in the expanded condition; and/or the first diameter may be less than 0.039 inches greater than a diameter of the cannula; and/or the second diameter may be between approximately 0.273 inches and approximately 0.280 inches; and/or the first balloon may be disposed a distance of between approximately 1mm (0.039 inches) and approximately 30mm (1.181 inches) from the leading end of the cannula; and/or the sheath may further include a hollow hub coupled to the cannula, and wherein the inflation port is defined in the hub; and/or the sheath may further include an inflation tube extending between the port and the first balloon, the inflation tube being in communication with the inflation port and an interior of the first balloon; and/or the sheath may further include a hemostasis seal disposed within the hub and configured to prevent fluid flow therethrough; and/or the inflation medium may be saline; and/or the inflation medium may be nitrogen; and/or the hub may further define a flush port for receiving a solution to remove air and lubricate the lumen; and/or the solution may include saline; and/or the sheath may further include a second balloon disposed on the cannula and spaced from the first balloon by a predetermined distance, the second balloon being configured and arranged to dilate from a collapsed condition to an expanded condition having a third diameter; and/or the third diameter of the second balloon may be substantially equal to the second diameter of the first balloon; and/or the predetermined distance between the first balloon and the second balloon may be substantially equal to at least one of a thickness of an ascending aorta and a thickness of a left ventricle.

In some embodiments, a method of delivering a collapsible prosthetic heart valve includes piercing an insertion location of a patient's body, partially inserting an introducer sheath into the patient's body, the insertion sheath having (i) a tubular cannula extending between a leading end and trailing end, the cannula defining a lumen therethrough, (ii) a first balloon disposed adjacent the leading end of the cannula, the first balloon being configured and arranged to dilate from a collapsed condition to an expanded condition, and (iii) an inflation port configured to receive an inflation medium to dilate the first balloon, and dilating the first balloon inside the patient's body to prevent movement of the introducer sheath in at least one direction.

In some examples, the insertion location may be the ascending aorta; and/or the insertion location may be the left ventricle; and/or the method may further include deflating the first balloon prior to removing the introducer sheath from the patient's body.

It will be appreciated that the various dependent claims and the features set forth therein can be combined in different ways than presented in the initial claims. It will also be appreciated that the features described in connection with individual embodiments may be shared with others of the described embodiments.

## Claims

1. An introducer sheath comprising:
a tubular cannula extending between a leading end and trailing end, the cannula defining a lumen therethrough;
a first balloon disposed adjacent the leading end of the cannula, the first balloon being configured and arranged to dilate from a collapsed condition to an expanded condition; and
an inflation port configured to receive an inflation medium to dilate the first balloon.

2. The introducer sheath of claim 1, further comprising a plurality of spaced markings disposed on the abluminal surface of the cannula.

3. The introducer sheath of claim 1, wherein the first balloon is configured to circumferentially dilate from a first diameter in the collapsed condition to a second diameter in the expanded condition.

4. The introducer sheath of claim 3, wherein the first diameter is less than 0.039 inches greater than a diameter of the cannula.

5. The introducer sheath of claim 3, wherein the second diameter is between approximately 0.273 inches and approximately 0.280 inches.

6. The introducer sheath of claim 1, wherein the first balloon is disposed a distance of between approximately 0.039 inches and approximately 1.181 inches from the leading end of the cannula.

7. The introducer sheath of claim 1, further comprising a hollow hub coupled to the cannula, and wherein the inflation port is defined in the hub.

8. The introducer sheath of claim 7, further comprising an inflation tube extending between the port and the first balloon, the inflation tube being in communication with the inflation port and an interior of the first balloon.

9. The introducer sheath of claim 1, further comprising a hemostasis seal disposed within the hub and configured to prevent fluid flow therethrough.

10. The introducer sheath of claim 1, wherein the inflation medium is selected from at least one of a saline or a contrast media.

11. The introducer sheath of claim 1, wherein the inflation medium is a bioabsorbable gas.

12. The introducer sheath of claim 1, wherein the hub further defines a flush port for receiving a solution to remove air and lubricate the lumen.

13. The introducer sheath of claim 12, wherein the solution comprises saline.

14. The introducer sheath of claim 1, further comprising a second balloon disposed on the cannula and spaced from the first balloon by a predetermined distance, the second balloon being configured and arranged to dilate from a collapsed condition to an expanded condition having a third diameter.

15. The introducer sheath of claim 14, wherein the third diameter of the second balloon is substantially equal to the second diameter of the first balloon.
